# EUROPEAN PATENT SPECIFICATION

(11) **EP 4 655 021 B1**
(45) Date of publication and mention of the grant of the patent: **25.11.2026**
(21) Application number: 23820964.7
(22) Date of filing: 13.12.2023
(51) Int. Cl.: A61M 5/142, A61M 5/172, A61M 5/31

(54) **WEARABLE MEDICAMENT DELIVERY DEVICE PACKAGE ASSEMBLY**
TRAGBARE VERPACKUNGSANORDNUNG FÜR MEDIKAMENTENABGABEVORRICHTUNG
ENSEMBLE EMBALLAGE DE DISPOSITIF D'ADMINISTRATION DE MÉDICAMENT PORTABLE

(30) Priority: 26.01.2023 US 202363441204 P; 26.01.2023 US 202363441206 P; 03.02.2023 US 202363443080 P; 03.02.2023 US 202363443086 P; 02.08.2023 EP 23189107
(43) Date of publication of application: 03.12.2025
(73) Proprietor: SHL Medical AG, 6302 Zug (CH)
(72) Inventor: MYRMAN, Mattias, 131 28 Nacka Strand (SE); COYNE III, Martin Michael, Boonton, New Jersey 07005 (US); SÄLL, Daniel, 131 28 Nacka Strand (SE)
(86) International application number: PCT/EP2023/085581
(87) International publication number: WO 2024/156429

(56) References cited:
- US-A- 5 868 710
- US-A1- 2010 145 276
- US-A1- 2018 008 781
- US-A1- 20260 144 941

## Description

### TECHNICAL FIELD

The present disclosure generally relates to medical devices for medicament administration.

### BACKGROUND

Infusion and injection are commonplace medical procedures used to deliver a wide variety of therapeutic medicines of interest for a variety of diseases. "Infusion," "injection," and "administration" may be used interchangeably, taking place by subcutaneous (SC), intramuscular (IM), intravenous (IV), enteral, or other routes, also terms used interchangeably. Administration route is based on a specific medication's pharmacokinetic (PK) profile, formulation components, approved regulatory labelling, individual clinical judgment, or clinical necessity.

The SC route is frequently used for administration of smaller volumes using prefilled syringes and autoinjectors. Biologic medicines are frequently administered via the SC route with these devices. SC administration is generally considered less invasive and more straightforward for patients, particularly compared to IV administration. Additionally, as physiologic uptake of medication is slower via the SC route, there is potential for improved tolerability compared to IV administration.

Given these significant advantages in safety, tolerability, and convenience, the pharmaceutical industry has invested heavily in transitioning formulations from IV to SC administration and medication administration from the clinic to the home setting. However, progress in large volume SC (LVSC) administration poses several issues that are related to the tolerability of a large volume of a single medication and the implications of this on pharmacokinetics when numerous LVSC medications are given sequentially or concurrently.

Certain biologic medications must be given in larger volumes that exceed the capacity of prefilled syringes and autoinjectors. For these medications, a larger volume device is used, such as e.g. an on-body injector (OBI, also known as a large volume infuser, "LVI," or bolus injector) including a drive unit (e.g. a motor driven pump or pressure drive) and an SC needle set. Such SC needle sets include a hollow-bore needle with sharpened point for percutaneous access to patient anatomy, and a tubing set for connection to the drive unit. Each needle may be used to deliver a specific volume of medication subcutaneously, limited by patient anatomy. For some medications of moderate volume infused relatively slowly, a single needle may suffice, as in the case of OBI devices. For other medications of comparatively larger volume, anywhere from 2-8 needles may be used, the number of needles generally increasing with increasing total medication volume and/or increasing infusion rate. Multiple needles may be connected with a splitter which allows a single medication reservoir, driven by a single drive unit, to split medication flow between the medication administration needles.

On-body injectors are wearable, as with an adhesive patch for the SC needle set, but particularly as medication volumes increase and the devices get larger, on-body injectors are increasingly uncomfortable, unwieldly, and/or interfere with daily activities of the user. With the increased size of the on-body injectors, the devices are also becoming increasingly immovable.

Some medicament devices are provided with a display for user feedback, and/or feedback to a caregiver of the user. For instance, the user and/or caregiver may monitor the progress of the infusion by looking at the display. However, with the increasing size of the medicament devices, various ways such devices may be worn on the body, or the increased immobility of the devices, the user may find it difficult to move the medicament device to see the display. In particular, a display that is arranged on the medicament device to provide information for a caregiver is typically not visible for a user during normal use. Also, if the user tries to move the medicament device with the aim to see the display, there is a risk of needle pull-out due to the movement and resulting tension on tubing or needle. Thus, there is a need for improvements of the medicament delivery device.

Published patent application US 2010/145276 A1 discloses a wearable infusion pump comprising a display.

### SUMMARY

In view of the above, there is a need for improved solutions of the medicament delivery devices, in particular for wearable medicament delivery devices.

There is hence in a fist aspect provided a wearable medicament delivery device package assembly comprising: a package body defining an interior reservoir for accommodating at least one medicament container, a pressure generating module configured to expel medicament from the medicament container, a display configured to present a message with symbols including letters and/or numbers, the display being configured to present the symbols with a default predetermined symbol representation, a control unit configured to receive a mirror indicating signal, and in response of receiving the mirror indicating signal, instruct the display to present the symbols of the message mirrored relative to the default predetermined symbol representation.

The wearable medicament delivery device package assembly can thus provide for a display that presents the symbols of the message mirrored in response of receiving a mirror indicating signal. Hereby, a user of the wearable medicament delivery device package assembly may read the message of the display in a mirror, and thus be presented the message of the display as if unmirrored. That is, as the mirrored message of the display is mirrored in the mirror, the mirrored message will be read by the user as if not being mirrored, i.e. seen as by the default predetermined symbol representation. Hereby, misinterpretations and erroneously read messages by the user can be avoided, or at least reduced. For example, some letters and numbers are legible in a mirror in both mirrored and unmirrored representation, e.g. "b" and "d" and "2" and "5", which may further increase the risk of confusion and misinterpretation when a user is trying to read and decode a message with default predetermined symbol representation through a mirror. Thus, by the first aspect, the risk of misinterpretations and erroneously read messages by the user is reduced, and an improved self-administration of medicament can be achieved. The letters of the message may be combined in text strings, and the numbers in number combinations.

The mirror indicating signal is to be understood as a signal indicating the presence of a mirror. The presence of the mirror may e.g. be constrained to the close proximity of the wearable medicament delivery device package, such as e.g. within 5 m of the wearable medicament delivery device package. The mirror indicating signal may be indicating the presence of a mirror internal of the wearable medicament delivery device package assembly, or of a mirror external of the wearable medicament delivery device package assembly.

In the present disclosure, the term "longitudinal", "longitudinally", "axially" or "axial" refer to a direction extending from a proximal end to a distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", or "circumferentially" refer to a circumference or a circumferential direction relative to an axis, typically a central axis extending in the direction of the longest extension of the device and/or component.

Similarly, "radial" or "radially" refer to a direction extending radially relative to the axis, and "rotation", "rotational" and "rotationally" refer to rotation relative to the axis.

According to one embodiment, the display is comprised in, or attached to, the package body.

According to one embodiment, the package body comprises an outer surface, wherein the display is comprised in, or attached to, the outer surface. Hereby, the display may present its message from the outer surface.

According to one embodiment, the display is arranged to face away from the package body. Hereby, a caregiver of the user can easily read the message on the display, or the user can ready the message through a mirror in case of that the control unit has received a mirror indicating signal, and thus instructed the display to present the symbols of the message mirrored.

The outer surface is to be understood as a surface facing in an external direction of the package body. Thus, with reference to a geometric centre of the package body, the outer surface is facing away from such geometric centre, contrary to an inner surface facing such geometric centre. Stated differently, the outer surface is facing away the interior reservoir. Thus, the outer surface is typically facing away from a user wearing the wearable medicament delivery device package assembly. Hereby, a caregiver of the user can easily read the message on the display, or the user can ready the message through a mirror as previously described. According to one embodiment, the outer surface may be provided with a cover, as described later.

According to one embodiment, the control unit is configured to, in the absence of receiving a mirror indicating signal, or in response to cessation of the mirror indicating signal, instruct the display to present the symbols of the message unmirrored. That is, the control unit is configured to instruct the display to present the symbols of the message by the default predetermined symbol representation. As an alternative, the control unit is configured to not send any instructions to the display in the absence of receiving a mirror indicating signal, or in response to cessation of the mirror indicating signal. Hereby, the display will present the symbols by the default predetermined symbol representation (i.e. unmirrored), as it has not received any signal instructing it otherwise.

According to one embodiment, the control unit is configured to, in response of receiving the mirror indicating signal, instruct the display to present the symbols of the message mirrored relative to the default predetermined symbol representation for a predefined time interval. The predefined time internal may e.g. be at least 10 seconds, such as for at least 20 seconds. After the predefined time interval ends, the display may be configured to again present the symbols by the default predetermined symbol representation (i.e. unmirrored).

According to one embodiment, the wearable medicament delivery device package assembly further comprises a transceiver configured to detect an external mirror of the wearable medicament delivery device package assembly, and in response of detecting the external mirror, transmit the mirror indicating signal to the control unit. Thus, the mirror indicating signal may be indicating the presence of an external mirror. Hereby, the user of the wearable medicament delivery device package assembly may read the message on the display through the external mirror as previously described, without manually instructing the display to present the message mirrored. That is, as the user is passing an external mirror detected by the transceiver, the symbols of the message will be presented mirrored on the display, so in case the user is looking at the mirror, he/she will be able to read the message through the mirror as previously described.

It should be understood that an external mirror is referring to a mirror being external of the wearable medicament delivery device package assembly.

The transceiver is typically configured to transmit a signal externally of the wearable medicament delivery device package assembly, and configured to receive a reflection of the signal. That is, as the signal is transmitted from the transceiver and is reflected on a surface, such as e.g. a mirror, the reflected signal is received by the transceiver.

The transceiver may be a single unit or be a combination of components/units at least comprising a transmitter or transmitting component and a receiver or a receiving component, e.g. a sensor (which could be combined with the transmitter). The transmitted signal, and/or the reflected signal may be simple (e.g. periodic pulses) or more complex (e.g. patterns of pulses) depending on the transmitting and receiving components, desired sensitivity (i.e., distinguishment) between mirroring and non-mirroring surface, and other factors. The transceiver may use visible or invisible light (e.g., infrared LED, laser, or light detection and ranging), audible or inaudible sound (e.g., ultrasonic source), other suitable methods, or combinations thereof.

According to one embodiment, the transceiver is configured to determine the degree of reflection of an external surface, wherein the external surface is detected as an external mirror in response to that the degree of reflection is above a predetermined threshold value. Hereby, the transceiver may distinguish between nonreflective external surfaces and reflective external surfaces, or between relatively low reflective external surfaces and relatively high reflective external surfaces, and only indicate the detection of an external mirror in case of the external surface being a reflective external surface, or a relatively high reflective external surface. That is, the transceiver can distinguish between highly reflective surfaces (e.g. a mirror) and comparatively less reflective surfaces (e.g. a wall). For example, the transceiver may be configured to, in response to receiving a reflected signal at a strength within a predetermined interval of the original strength (of the transmitted signal), indicate the detection of an external mirror, while also optionally distinguishing said mirror from a less reflective surface, such as a window.

It should be understood that the external mirror should be broadly defined, and need not to correspond to an actual mirror. For example, a surface which is highly reflective, i.e. mirror-like, may be defined as an external mirror by the transceiver. Moreover, the reflectance may be configured to a desired level within the control or transceiver based on, for example, the expected user of the apparatus (e.g., patient, lay caregiver, or healthcare provider), or the expected use environment (e.g., home or clinic). Additionally, the reflectance may be adaptively configured corresponding to ambient lighting conditions, as during use in daylight versus lower lighting at night.

According to one embodiment, the transceiver may be configured to compare the intensity, modulation, or phase of transmitted and received signals. Hereby, "false positives" that could arise from flat, moderately reflective surfaces, such as windows or wall tiles can be eliminated, or at least reduced.

According to one embodiment, the transceiver is configured to continuously or periodically scan the surroundings for external mirrors, or the transceiver is configured to scan the surroundings for external mirrors in response to receiving an external scanning signal, e.g. provided by a user of the wearable medicament delivery device package assembly.

According to one embodiment, the wearable medicament delivery device package assembly further comprises a flip cover having an internal mirror, the flip cover being arrangeable in a folded position in which the flip cover is arranged to cover the display, and being arrangeable in an unfolded position in which the flip cover is arranged to at least partly uncover the display such that at least a part of the internal mirror is distant from, and facing, the display, wherein the control unit is configured to identify the unfolded position of the flip cover as a mirror indicating signal. Thus, the mirror indicating signal may be indicating the presence of an internal mirror.

It should be understood that an internal mirror is referring to a mirror being internal of, or integrated into, the wearable medicament delivery device package assembly.

Typically, in the folded position, the internal mirror fully faces the display, e.g. at no angle, so that the internal mirror and display are parallel. Thus, the internal mirror and the flip cover may function as a protective lid for the display. Hereby, buttons and similar user interface (UI) elements are encapsulated and may not be pressed unintentionally.

In the unfolded position, at least a part of the internal mirror is distant from, and facing, the display, so that the internal mirror and display are non-parallel. For example, the display and the internal mirror may form a "V"-shape, in which the left leg of the "V" which faces the right leg of the "V" makes up the display, and the right leg of the "V" which faces the left leg of the "V" makes up the internal mirror. Typically, the flip cover is configured to move from the folded position into the unfolded position.

According to one embodiment, the flip cover is, in the unfolded position, arranged relative the display with an angle of between 10° and 170°. Hereby, the internal mirror may reflect the presented message of the display in an advantageous angle.

According to one embodiment, the flip cover extends from a first end defined by a first end axis, to a second end defined by a second end axis, wherein the flip cover is attached to the package body at the first end such that the flip cover is foldable relative to the package body by rotating about the first end axis. Hereby, the previously "V"-shape can advantageously be achieved. Thus, in the unfolded position, the first end will be adjacent, and connected to, the package body, and the second end will be spaced apart from the package body.

According to one embodiment, the flip cover comprises a planar base portion extending from the first end to the second end, the planar base portion having a first planar surface and a second planar surface arranged on the opposite side of planar base portion as compared to the first planar surface, wherein the internal mirror is comprised in, or attached to, the first planar surface. The first planar surface is to be understood as a surface facing towards the package body, and the display. Thus, with reference to a geometric centre of the package body, the first planar surface is facing towards such geometric centre, contrary to the second planar surface facing away from such geometric centre. Stated differently, the first planar surface is facing towards the interior reservoir. Thus, the first planar surface is typically facing towards a user wearing the wearable medicament delivery device package assembly. Hereby, the user can ready the message through the internal mirror as previously described.

According to one embodiment, the internal mirror is provided as a mirrored coating on the first planar surface. Hereby, an efficient means for providing the internal mirror is provided. The mirrored coating may e.g. be provided as a separate film, be molded into the first planar surface as a two-shot operation or via an in-mold decoration, or be provided by PVD/CVD or other suitable methods. The mirrored coating may e.g. be a half-slivered film.

According to one embodiment, the internal mirror is a one-way mirror and the planar base portion is at least partly transparent. Hereby, the display is visible for a caregiver when the flip cover is arranged in the folded position. That is, a caregiver can view the display through the flip cover and the at least partly transparent planar base portion and the one-way mirror. Thus, the one-way mirror is to be understood as being mirroring when being viewed from one direction, but transparent when being view from another, opposite, direction.

According to one embodiment, the internal mirror is a concave mirror. The concave mirror optically acts as a convex lens. Hence, the concave mirror enlarges the mirrored messages from the display. Moreover, by using a concave mirror, it is possible for a user to focus on the display, even though the distance to the display may otherwise be too close for proper focusing. An additional advantage of a concave mirror is that presentation from the display is enlarged at a low cost. The concave mirror is preferably adapted so that the eyes of a user is positioned within the focal length of the internal mirror.

According to one embodiment, the concave mirror is adapted to compensate for a varying distance to the display, due to the angle of the internal mirror relative the eyes of the user. Hereby, distortion of the display (such as e.g. a larger upper part compared to a lower part) may be eliminated, or at least reduced.

According to one embodiment, the wearable medicament delivery device package assembly further comprises a wearable strap attached to the package body, wherein the wearable strap is configured to be worn around the user such that the package body is arranged at a fixed position relative the user. Hereby, even though the package body, and hence the display, are fixed in position relative the user, the user may properly read the message of the display without the need to re-arrange or move the wearable medicament delivery device package assembly with the associated risks of needle pull-out due to the movement and resulting tension on tubing or needle.

According to one embodiment, the wearable strap is arranged such that the package body, and the display, are arranged in a position in which they cannot be rotated. According to one embodiment, the wearable strap is configured for being worn around the chest or an upper arm of the user. The wearable strap may e.g. be a harness, a reverse back-pack, a shoulder strap, a belt or the like.

According to one embodiment, the display is directly, or indirectly, attached to the wearable strap such that the display is arranged to face away from the user when the wearable medicament delivery device package assembly is worn by the user. As previously described, the display is typically comprised, or attached to, the package body.

According to one embodiment, the default predetermined symbol representation includes horizontal orientation of the symbols. That is, with reference to the display, the symbols are presented horizontally, i.e. the orientation of individual letters or numbers (vertical) is orthogonal to the spatial arrangement (horizontal) of the letters or numbers within a word or number combination. It should be understood that the default predetermined symbol representation typically excludes mirrored symbols.

According to one embodiment, the wearable medicament delivery device package assembly further comprises at least a delivery tube for providing medicament to an injection assembly, the delivery tube being fluidly connected to the associated medicament container.

According to one embodiment, the wearable medicament delivery device package assembly further comprises an injection assembly for medicament administration comprising at least a medicament delivery member fluidly connected to the medicament container via the delivery tube for a predefined medicament administration. The medicament delivery member is preferably a needle, for example one of a right-angle needle, a straight needle, or a soft cannula.

With predefined medicament administration is meant that a predefined type of drug/medicament is to be administered at a predefined rate, for a predefined amount of time. The target patient group may also be predefined.

According to one embodiment, the pressure generating module comprises a pump being fluidly connected to the medicament container and configured to provide a suction force for a medicament inside the medicament container. Thus, an efficient means for expelling medicament from the medicament containers is provided. Thus, a suction force is achieved by the pump, e.g. by achieving a vacuum, or an under pressure (suppression), as compared to the pressure within the medicament container. Hereby, medicament contained within the medicament container is sucked out of the medicament container. The pump may e.g. be arranged integrated into the delivery tube, or be arranged at an end portion of the delivery tube.

According to one embodiment, the pressure generating module comprises a pressure drive configured to provide a pressure force for a medicament inside the medicament container. Thus, an efficient means for expelling medicament from the medicament container is provided. Thus, a pressure force is achieved by the pressure drive, e.g. by achieving pressure difference between outside and inside of the medicament container. Hereby, medicament contained within the medicament container is pressed out of the medicament container.

According to one embodiment, the wearable medicament delivery device package assembly comprises a battery configured to power the pressure generating module.

According to one embodiment, the wearable medicament delivery device package assembly is configured to be wearable near-body the user. That is, the wearable medicament delivery device package assembly is wearable over or under the clothing of the user, without being directly attached to the user's body, e.g. without being directly attached to the user's skin. In such a configuration, the needle assembly is placed onto the skin to deliver medication to the patient, and the associated tubing set connecting the wearable medicament delivery device package is routed as desired by the user.

According to one embodiment, the package body is a reusable package body. A reusable package body have advantages for sustainability and user convenience.

According to one embodiment, the wearable medicament delivery device package assembly comprises a base plate attached to the package body, the base plate comprising a plurality of connecting slots separated from each other by a spacing. Thus, for embodiments in which the package body is configured to house a plurality of medicament containers and a plurality of pressure generating modules, each one of the medicament containers being coupled to a corresponding pressure generating module, each one of the connecting slots is associated with a pressure generating module being releasably attached therein.

Generally, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 shows an example of a wearable medicament delivery device package assembly;
Fig. 2 shows an example of a pressure generating module of the wearable medicament delivery device package assembly of Fig. 1;
Fig. 3 shows a package body of the wearable medicament delivery device package assembly 1 of Fig. 1;
Figs. 4A-4B show the display 40 of the wearable medicament delivery device package assembly 1 of Fig. 1;
Fig. 5 shows another example of a wearable medicament delivery device package assembly;
Fig. 6 shows another example of a wearable medicament delivery device package assembly;
Figs. 7A-7B show details of the wearable medicament delivery device package assembly of Fig. 6;
Fig. 7C shows an example of an internal mirror for use with the wearable medicament delivery device package assembly of Fig. 6; and
Fig. 8 shows an alternative embodiment of a wearable medicament delivery device package assembly.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

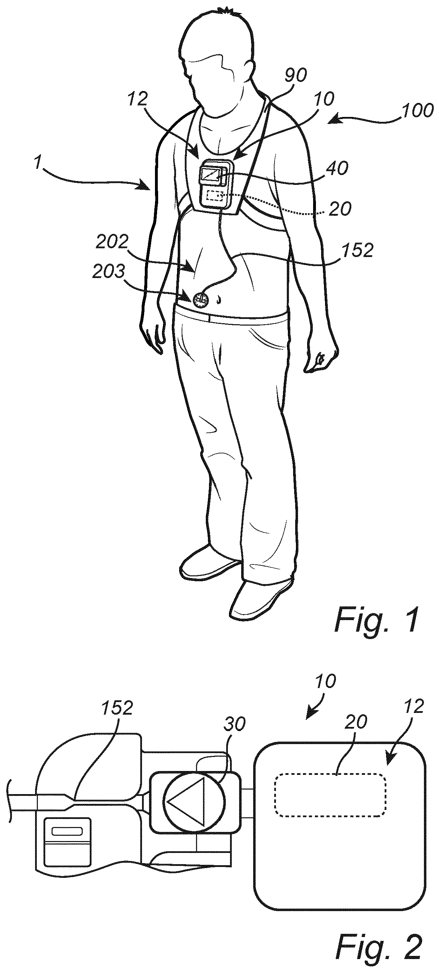

Fig. 1 shows a first example of wearable medicament delivery device package assembly 1 according to the present disclosure, and Fig. 2 shows details of the wearable medicament delivery device package assembly 1 of Fig. 1.

The wearable medicament delivery device package assembly 1 comprises a package body 10 defining an interior reservoir 12. The interior reservoir 12 is configured to accommodate at least one medicament container 20.

As seen in Fig. 2, the wearable medicament delivery device package assembly 1 comprises a pressure generating module 30, here in the form of a pump, configured to expel medicament from the medicament container 20. Moreover, a delivery tube 152 for providing medicament to an injection assembly 202 is attached to the outlet of the pressure generating module 30, the delivery tube being 152 thus being fluidly connected to the medicament container 20. Thus, the injection assembly 202 may be used for a medicament administration. The delivery tube 152 and the injection assembly 202 may be comprised in the wearable medicament delivery device package assembly 1.

The injection assembly 202 may comprise a medicament delivery member 203, typically in the form a first needle, fluidly connected to the medicament container 20 via the first delivery tube 152 for a predefined medicament administration. It should be noted that the wearable medicament delivery device package assembly 1 may comprise more than one medicament container held within the interior reservoir 12 of the package body 10, as well as more than pressure generating module, more than one delivery tube and more than one medicament delivery member. For example, the injection assembly 202 may further comprises a second medicament delivery member, typically in the form of a second needle, fluidly connected to a second medicament container via a second delivery tube and a second pressure generating module for a second predefined medicament administration. Hereby, the wearable medicament delivery device package assembly 1 may provide multiple medicament administrations, e.g. multiple (and possibly different) medications, or large volumes of the same medicament may be delivered simultaneously.

As shown in Fig. 1, the medicament delivery device package assembly is a wearable medicament delivery device package assembly 1. In the present embodiment, the user 100 wear the wearable medicament delivery device package assembly 1 slung across the body of the user 100. The user 100 may be referred to as a patient. The wearable medicament delivery device package assembly 1 comprises a wearable strap 90 attached to the package body 10. The wearable strap 90 is configured to be worn around the user 100 such that the package body is arranged at a fixed position relative the user 100. By the wearable strap 90, the wearable medicament delivery device package assembly 1 can be worn near-body the user, over or under the clothing of the patent, without being directly attached to the user's body, e.g. without being directly attached to the user's skin.

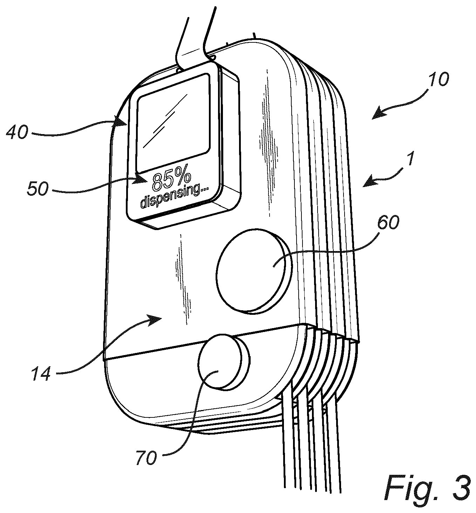

The wearable medicament delivery device package assembly 1 of Fig. 1 comprises a display 40, which will be further described with reference to Fig. 3.

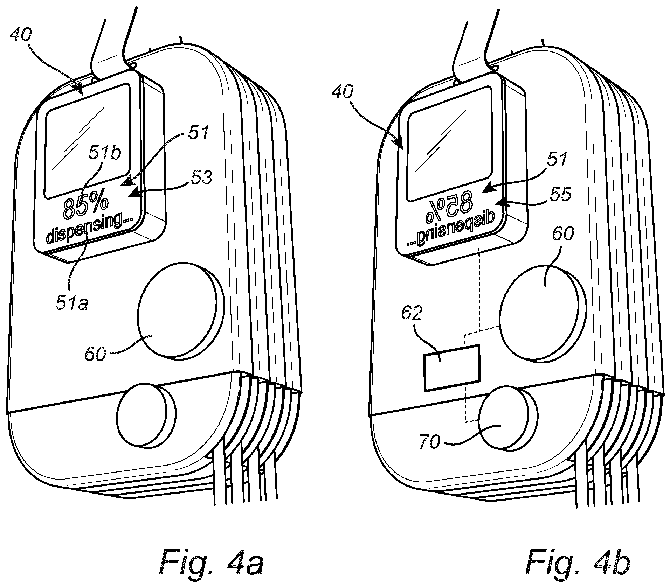

Fig. 3 shows the package body 10 of the wearable medicament delivery device package assembly 1 of Fig. 1 in greater detail, and Figs. 4A-4B show the display 40 of the wearable medicament delivery device package assembly 1 in greater detail.

The package body 10 comprises an outer surface 14. The outer surface 14 is facing in an external direction of the package body 10, and typically facing away from the user 100 wearing the wearable medicament delivery device package assembly 1, as seen in Fig. 1. The display 40 is in the embodiment of Fig. 3 comprised in, or attached to, the outer surface 14. Thus, the display 40 is arranged to face away from the package body 10, and to face away from a user 100 when the wearable medicament delivery device package assembly 1 is worn by the user 100 as shown in Fig. 1. However, it should be noted that the display 40 need not to be comprised in, or attached to, the package body 10, and thus indirectly attached to the wearable strap 90. Instead, the display 40 may be directly attached to the wearable strap 90, or another component of the wearable medicament delivery device package assembly 1. In either way, the display 40 is typically arranged to face away from the user 100 when the wearable medicament delivery device package assembly 1 is worn by the user 100.

The display 40 is configured to present a message 50, as better shown in Fig. 4A. The message 50 comprises symbols 51 including at least one of letters 51a and numbers 51b. In the example of Fig. 4A, the message includes letters 51, numbers 51b as well as other symbols, here as the symbol of percentage "%".

Typically, the letters 51a of the message 50 are combined in text strings, and the numbers 51b of the message 50 are combined in number combinations. For example, the message 50 may include dose progress, end of dose, time left of the medicament administration, injection status confirmation and/or error states.

The display 40 is configured to present the symbols 51 with a default predetermined symbol representation 53. By the default predetermined symbol representation 53, the symbols 51 are presented in their default mode (or normal mode) meaning that they are at least unmirrored. Thus, if the user 100 would try to read the message 50 of the display 40 in a mirror, there is a risk that the user 100 will misinterpret the message 50 or erroneously read the message 50, in particular as at least some letters and numbers are legible in a mirror in both mirrored and unmarried representation, e.g. "b" and "d" and "2" and "5", making the decoding of the message 50 through the mirror even more complex.

The wearable medicament delivery device package assembly 1 comprises a control unit 60, e.g. comprised in the package body 10. The control unit 60 is configured to receive a mirror indicating signal 62, and in response of receiving the mirror indicating signal 62, instruct the display 40 to present the symbols 51 of the message 50 mirrored 55 relative to the default predetermined symbol representation 53, as shown in Fig. 4B. Thus, if the user 100 would try to read the message 50 of the display 40 in a mirror, the user 100 will be presented the message 50 of the display 40 as if unmirrored. That is, as the mirrored message 55 of the display 40 is mirrored in the mirror, the mirrored message 55 will be read by the user 100 as if not being mirrored, i.e. seen as by the default predetermined symbol representation 53 shown in Fig. 4A. Hereby, misinterpretations and erroneously read messages by the user 100 can be avoided, or at least reduced, and an improved self-administration of medicament can be achieved as the user 100 may read and interpret the message 50 in an improved manner. In other words, the user 100 does not need to decode the message 50 in the mirror, which would be the case if the symbols 51 were presented with its default predetermined symbol representation 53, possibly resulting in a erroneously self-administration of medicament.

In both of Figs. 4A and 4B, the symbols 51 are presented horizontally, i.e. the orientation of individual letters 51a and numbers 51b is vertical and orthogonal to the spatial arrangement of the letters and numbers which is horizontal.

The wearable medicament delivery device package assembly 1 may further comprise a transceiver 70 connected to the control unit 60. The transceiver 70 is configured to detect an external mirror of the wearable medicament delivery device package assembly 1, and in response of detecting the external mirror, transmit the mirror indicating signal 62 to the control unit 60, as shown in Fig. 4B. The transceiver 70 may be configured to continuously or periodically scan the surroundings for external mirrors.

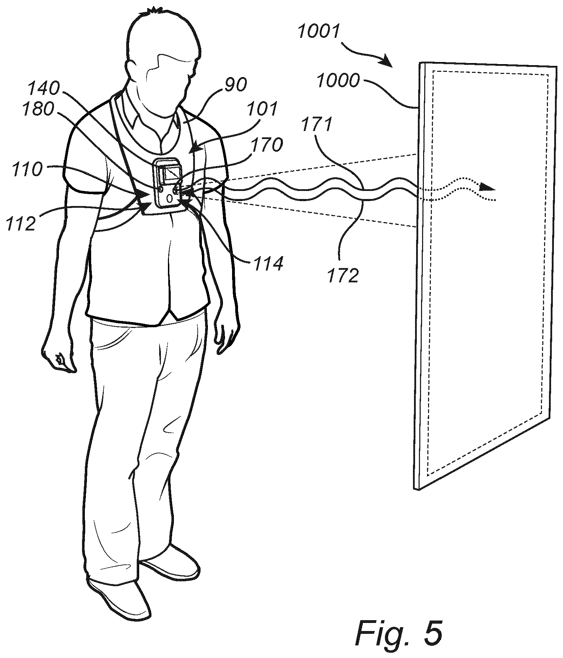

However, it should be noted that the transceiver 70 in the embodiments of Fig. 1 and Figs. 4A-4B is optional, and that the mirror indicating signal 62 may be achieved by other means than the transceiver detecting an external mirror, as will be described later. For example, and according to one embodiment, the user 100 can manually provide the mirror indicating signal 62 by e.g. interacting with the wearable medicament delivery device package assembly 1, e.g. by means of a push button. Thus, if the user 100 notifies the presence of an external mirror, he/she may push the push button resulting in that the mirror indicating signal 62 is sent to, and received by, the control unit 60. For example, the push button 180 described with reference to Fig. 5 may be used for this purpose (i.e. without the functionality related to the transceiver).

Turning to Fig. 5 showing another embodiment of a wearable medicament delivery device package assembly 101 comprising a transceiver 170. As for the wearable medicament delivery device package assembly 1 in the embodiment of Fig. 1, the wearable medicament delivery device package assembly 101 comprises a package body 110 defining an interior reservoir 112 for accommodating at least one medicament container (not shown).

Typically, the package body 110 comprises at least one pressure generating module, at least one delivery tube and at least one injection assembly as described for the wearable medicament delivery device package assembly 1 in the embodiment of Fig. 1, thus not repeated here again. As also shown in Fig. 5, the wearable medicament delivery device package assembly 101 comprises a wearable strap 90 attached to the package body 110, as described for the wearable medicament delivery device package assembly 1 in the embodiment of Fig. 1, and thus not repeated here again.

Moreover, the wearable medicament delivery device package assembly 101 of Fig. 5 comprises a display 140 comprised in an outer surface 114 of the package body 110, and thus facing away from the user 100. The display 140 may be the same, or corresponding, to the display 40 described with reference to Fig. 3, and Figs. 4A-4B. The wearable medicament delivery device package assembly 101 typically comprises a control unit (not shown) operable in the same way as described with reference to Figs. 4A-4B.

As shown in Fig. 5, the wearable medicament delivery device package assembly 101 comprises a transceiver 170 configured to detect an external mirror 1000 of the wearable medicament delivery device package assembly 1.

The transceiver 170 is in the embodiment of Fig. 5 comprised in the outer surface 114 of the package body 110. The transceiver 170 is configured to transmit a signal 171 externally of the wearable medicament delivery device package assembly 1, and configured to receive a reflected signal 172 of the transmitted signal 171. That is, as the transmitted signal 171 is sent from the transceiver 170, encounters an external surface 1001 and is reflected back as a reflected signal 172. The transceiver 170 is configured to receive the reflected signal 172. Hereby, the transmitted signal 171 can be compared with the reflected signal 171, and the characteristics of the external surface 1001 at which the signal was reflected on can be evaluated.

For example, the transceiver 170 may be configured to determine the degree of reflection of the external surface 1001. The external surface 1001 may e.g. be detected as an external mirror 1000 in response to that the degree of reflection is above a predetermined threshold value. Typically, the control unit previously described can be used for such signal processing and evaluation.

The wearable medicament delivery device package assembly 101 comprises a push button 180. The push button 180 is configured to be pushed by the user 100 and may be used for various purposes. According to one embodiment, the push button 180 is used for manually triggering the mirror indicating signal 62 as previously described. According to another embodiment, the push button 180 is used to trigger the transceiver 170 to scan the surroundings for external mirrors 1000. Thus, when the user 100 pushes the push button 180, an external scanning signal is sent to the transceiver 170, wherein the transceiver begins with scanning the surroundings for external mirrors 1000.

In response of detecting an external mirror 1000, the mirror indicating signal 62 is sent to the control unit 60 as described with reference to Fig. 4B, and the user may read the message 50 in the external mirror 100 as previously described.

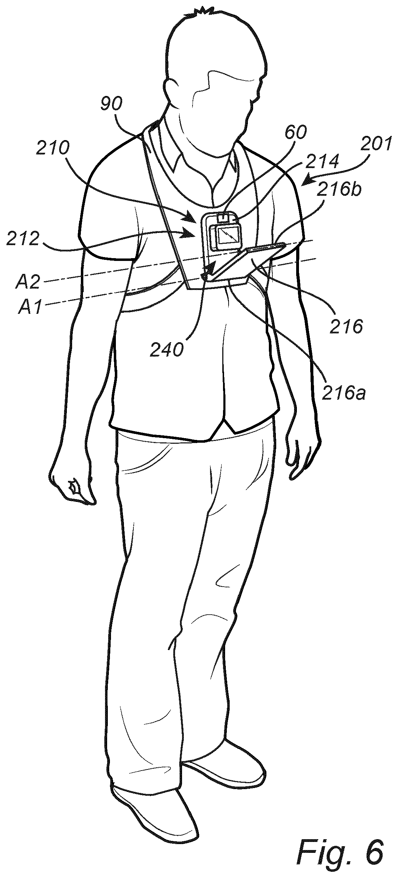

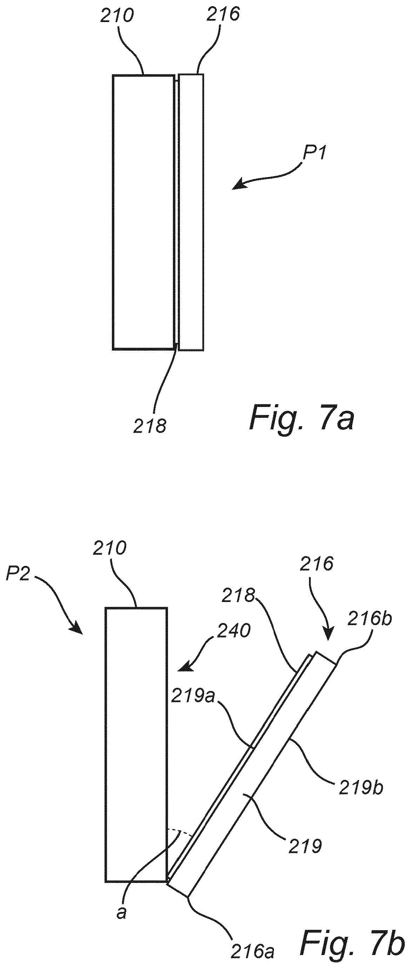

Fig. 6 shows another embodiment of a wearable medicament delivery device package assembly 201, and Figs. 7A-7B show details of the wearable medicament delivery device package assembly 201 in greater detail.

As for the wearable medicament delivery device package assembly 1 in the embodiment of Fig. 1, the wearable medicament delivery device package assembly 201 comprises a package body 210 defining an interior reservoir 212 for accommodating at least one medicament container (not shown).

Typically, the package body 210 comprises at least one pressure generating module, at least one delivery tube and at least one injection assembly as described for the wearable medicament delivery device package assembly 1 in the embodiment of Fig. 1, thus not repeated here again. As also shown in Fig. 6, the wearable medicament delivery device package assembly 201 comprises a wearable strap 90 attached to the package body 210, as described for the wearable medicament delivery device package assembly 1 in the embodiment of Fig. 1, and thus not repeated here again.

Moreover, the wearable medicament delivery device package assembly 201 of Fig. 6 comprises a display 240 comprised in an outer surface 214 of the package body 210, and thus facing away from the user 100. The display 240 may be the same, or corresponding, to the display 40 described with reference to Fig. 3 and Figs. 4A-4B. The wearable medicament delivery device package assembly 201 typically comprises a control unit 60 operable in the same way as described with reference to Figs. 4A-4B.

As shown in Fig. 6, the wearable medicament delivery device package assembly 201 comprises a flip cover 216 having an internal mirror 218, better shown in Fig. 7B. The flip cover 216 is arrangeable in a folded position P1, shown in Fig. 7A, in which the flip cover 216 is arranged to cover the display 240, and is arrangeable in an unfolded position P2, shown in Fig. 7B, in which the flip cover 216 is arranged to at least partly uncover the display 240 such that at least a part of the internal mirror 218 is distant from, and facing, the display 240.

Thus, in the unfolded position P2, the user 100 may look at the internal mirror 218 and see the reflection of the display 240 therein.

The control unit 60 in the embodiment of Fig. 6 is configured to identify the unfolded position P2 of the flip cover 216 as a mirror indicating signal 62. Thus, as described with reference to Fig. 4B, the control unit 60 is configured to receive the mirror indicating signal 62, but here as a response to that the control unit 60 identifies the unfolded position P2. Subsequently, the control unit 60 is configured to, in response of receiving the mirror indicating signal 62, instruct the display 240 to present the symbols 51 of the message 50 mirrored 55 relative to the default predetermined symbol representation 53, as previously described. Thus, if the user 100 try to read the message 50 of the display 40 in the internal mirror 218, the user 100 will be presented the message 50 of the display 240 as if unmirrored. That is, as the mirrored message 55 of the display 240 is mirrored in the internal mirror 218, the mirrored message 55 will be read by the user 100 as if not being mirrored, i.e. as seen by the default predetermined symbol representation 53. Thus, the wearable medicament delivery device package assembly 201 does not use a transceiver for generating the mirror indicating signal 62.

As shown in Fig. 7B, the flip cover 216 is, in the unfolded position P2, arranged tilted relative the display with an angle a of approximately 45°. However, any value of between 10° and 170° is feasible, as long as the user may read the message 50 of the display 240 in the internal mirror 218.

In more detail, the flip cover 216 comprises a planar base portion 219 extending from a first end defined by a first end axis A1, to a second end 216b defined by a second end axis A2. The flip cover 216, and the planar base portion 219, is attached to the package body 210 at the first end 216a, such that the flip cover 216 and the planar based portion 219 is foldable relative to the package body 210 by rotating about the first end axis A1, as shown when comparing Fig. 7A with Fig. 7B.

The planar base portion 219 has a first planar surface 219a and a second planar surface 219b arranged on the opposite side of planar base portion 219 as compared to the first planar surface 219a. The first planar surface 219a is facing the package body 210 and the display 240 as well as the user 100 wearing the wearable medicament delivery device package assembly 201. The second planar surface 219b is facing away from the package body 210 and the user 100.

The internal mirror 219 is comprised in, or attached to the first planar surface 219a. Hereby, the user 100 can ready the message 50 through the internal mirror 219 as previously described.

As seen in Fig. 7B, the internal mirror 218 may be provided as a mirrored coating on the first planar surface 219a. Thus, efficient means for providing the internal mirror onto the flip cover 216 is achieved.

The internal mirror 218 is typically a one-way mirror. That is, the internal mirror is configured to be mirroring when being viewed from one direction, typically from the package body 210, or user 100, towards the internal mirror 218, but transparent when being view from another, opposite, direction, typically from externally of the wearable medicament delivery device package assembly 201 and towards the internal mirror 218. Moreover, the planar base portion 219 is preferably at least partly transparent. Hereby, the display 240 is visible for a caregiver when the flip cover 216 is arranged in the folded position P1. That is, a caregiver can view the display 240 through the at least partly transparent planar base portion 219 and through the one-way internal mirror 218.

According to one embodiment, the wearable medicament delivery device package assembly 201 comprises adaptive illumination of the display 240. For example, in response to that the flip cover 216 is arranged in its folded position P1, i.e. the position in which a caregiver can view the display 240 through the at least partly transparent planar base portion 219 and through the one-way internal mirror 218, the adapted illumination may be used, or increased, to compensate for reduced transparency. Thus, the adapted illumination may provide for illumination intensity which corresponds to the position of the flip cover 216. For example, in the unfolded position P2, the adapted illumination may be turned off, or decreased, as there is a lesser need for extra illumination.

The control unit 60 may be configured to, in the absence of receiving a mirror indicating signal 62, or in response to cessation of the mirror indicating signal 62, or in response to that the flip cover 216 is arranged in the folded position P1, instruct the display 240 to present the symbols 51 of the message 50 unmirrored. That is, the control unit 60 may be configured to instruct the display 240 to present the symbols 51 of the message 50 by the default predetermined symbol representation 53.

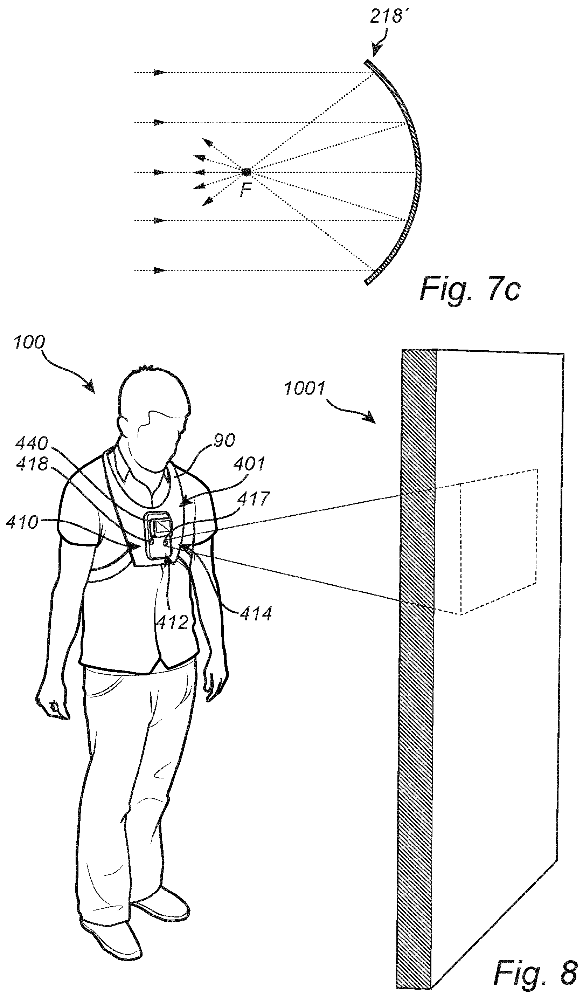

In Fig. 7C, an embodiment of the internal mirror 218' is shown. The internal mirror 218' may replace the internal mirror 218 described with reference to Figs. 7A-7B. As shown in Fig. 7C, the internal mirror 218' is a concave mirror. Hereby, the concave internal mirror 218' enlarges the mirrored messages 55 from the display 240. The concave internal mirror 218' is preferably adapted so that the eyes of a user is positioned within the focal length, defined by the focal point F, of the internal mirror 218'.

In Fig. 8, an alternative embodiment of a wearable medicament delivery device package assembly 301 is shown. As for the wearable medicament delivery device package assembly 1 in the embodiment of Fig. 1, the wearable medicament delivery device package assembly 401 comprises a package body 410 defining an interior reservoir 412 for accommodating at least one medicament container (not shown). Typically, the package body 410 comprises at least one pressure generating module, at least one delivery tube and at least one injection assembly as described for the wearable medicament delivery device package assembly 1 in the embodiment of Fig. 1, thus not repeated here again. As also shown in Fig. 8, the wearable medicament delivery device package assembly 401 comprises a wearable strap 90 attached to the package body 410, as described for the wearable medicament delivery device package assembly 1 in the embodiment of Fig. 1, and thus not repeated here again.

Moreover, the wearable medicament delivery device package assembly 401 of Fig. 8 comprises a display 440 comprised in an outer surface 414 of the package body 410, and thus facing away from the user 100. The display 440 may be the same, or corresponding, to the display 40 described with reference to Fig. 4A, possibly with the exception that the display is not configured to present the symbols of the message mirrored relative to the default predetermined symbol representation.

As shown in Fig. 8, the wearable medicament delivery device package assembly 401 comprises a projector 417 configured to present the message of the display 440 on an external surface 1001, such as e.g. a white wall. Thus, the projector 417 is configured to visualize information from the wearable medicament delivery device package assembly 401 to an external surface 1011, such as a wall or ceiling. The projected information may be activated when the user 100 presses a push button 418, as the previously described. Hereby, the user 100 may be provided with large and easy to read information from the message using a relatively small display that is not visible during normal use (as the wearable medicament delivery device package assembly 401 is arranged in a fixed position relative to the user 100).

The package body, and the medicament containers, may take on numerous different outer shapes, depending on the design, the pressure generating modules (providing a pressure force or a suction force), number of medicament delivery members, desired injection rotation schedule between injections, the volume of medicament to be infused at a given site through a given delivery member, nature of the medicines themselves, medication regimen, medication administration sequence(s), a desire to prevent mixing of medications at their respective injection sites, or based on results from pharmacokinetic/pharmacodynamic modelling or human clinical trial(s) of one or more medicaments.

The wearable medicament delivery device package assemblies described herein can be used for the treatment and/or prophylaxis of one or more of many different types of disorders.

Exemplary disorders include, but are not limited to: rheumatoid arthritis, inflammatory bowel diseases (e.g. Crohn's disease and ulcerative colitis), hypercholesterolaemia and/or dyslipidemia, cardiovascular disease, diabetes (e.g. type 1 or 2 diabetes), psoriasis, psoriatic arthritis, spondyloarthritis, hidradenitis suppurativa, Sjögren's syndrome, migraine, cluster headache, multiple sclerosis, neuromyelitis optica spectrum disorder, anaemia, thalassemia, paroxysmal nocturnal hemoglobinuria, hemolytic anaemia, hereditary angioedema, systemic lupus erythematosus, lupus nephritis, myasthenia gravis, Behçet's disease, hemophagocytic lymphohistiocytosis, atopic dermatitis, retinal diseases (e.g., age-related macular degeneration, diabetic macular edema), uveitis, infectious diseases, bone diseases (e.g., osteoporosis, osteopenia), asthma, chronic obstructive pulmonary disease, thyroid eye disease, nasal polyps, transplant, acute hypoglycaemia, obesity, anaphylaxis, allergies, sickle cell disease, Alzheimer's disease, Parkinson's disease, dementia with Lewy bodies, systemic infusion reactions, immunoglobulin E (IgE)-mediated hypersensitivity reactions, cytokine release syndrome, immune deficiencies (e.g., primary immunodeficiency, chronic inflammatory demyelinating polyneuropathy), enzyme deficiencies (e.g., Pompe disease, Fabry disease, Gaucher disease), growth factor deficiencies, hormone deficiencies, coagulation disorders (e.g., hemophilia, von Willebrand disease, Factor V Leiden), and cancer.

Exemplary types of drugs that could be included in the wearable medicament delivery device package assemblies described herein include, but are not limited to, small molecules, hormones, cytokines, blood products, enzymes, vaccines, anticoagulants, immunosuppressants, antibodies, antibody-drug conjugates, neutralizing antibodies, reversal agents, radioligand therapies, radioisotopes and/or nuclear medicines, diagnostic agents, bispecific antibodies, proteins, fusion proteins, peptibodies, polypeptides, pegylated proteins, protein fragments, nucleotides, protein analogues, protein variants, protein precursors, protein derivatives, chimeric antigen receptor T cell therapies, cell or gene therapies, oncolytic viruses, or immunotherapies.

Exemplary drugs that could be included in the wearable medicament delivery device package assemblies described herein include, but are not limited to, immuno-oncology or bio-oncology medications such as immune checkpoints, cytokines, chemokines, clusters of differentiation, interleukins, integrins, growth factors, coagulation factors, enzymes, enzyme inhibitors, retinoids, steroids, signaling proteins, pro-apoptotic proteins, anti-apoptotic proteins, T-cell receptors, B-cell receptors, or costimulatory proteins.

Exemplary drugs that could be included in the wearable medicament delivery device package assemblies described herein include, but are not limited to, those exhibiting a proposed mechanism of action, such as human epidermal growth factor receptor 2 (HER-2) receptor modulators, interleukin (IL) modulators, interferon (IFN) modulators, complement modulators, glucagon-like peptide-1 (GLP-1) modulators, glucose-dependent insulinotropic polypeptide (GIP) modulators, cluster of differentiation 38 (CD38) modulators, cluster of differentiation 22 (CD22) modulators, C1 esterase modulators, bradykinin modulators, C-C chemokine receptor type 4 (CCR4) modulators, vascular endothelial growth factor (VEGF) modulators, B-cell activating factor (BAFF), P-selectin modulators, neonatal Fc receptor (FcRn) modulators, calcitonin gene-related peptide (CGRP) modulators, epidermal growth factor receptor (EGFR) modulators, cluster of differentiation 79B (CD79B) modulators, tumor-associated calcium signal transducer 2 (Trop-2) modulators, cluster of differentiation 52 (CD52) modulators, B-cell maturation antigen (BCMA) modulators, enzyme modulators, platelet-derived growth factor receptor A (PDGFRA) modulators, cluster of differentiation 319 (CD319 or SLAMF7) modulators, programmed cell death protein 1 and programmed death-ligand 1 (PD-1/PD-L1) inhibitors/modulators, B-lymphocyte antigen cluster of differentiation 19 (CD19) inhibitors, B-lymphocyte antigen cluster of differentiation 20 (CD20) modulators, cluster of differentiation 3 (CD3) modulators, cytotoxic T-lymphocyte-associated protein 4 (CTLA-4) inhibitors, T-cell immunoglobulin and mucin-domain containing-3 (TIM-3) modulators, T cell immunoreceptor with Ig and ITIM domains (TIGIT) modulators, V-domain Ig suppressor of T cell activation (VISTA) modulators, indoleamine 2,3-dioxygenase (IDO or INDO) modulators, poliovirus receptor-related immunoglobulin domain-containing protein (PVRIG) modulators, lymphocyte-activation gene 3 (LAG3; also known as cluster of differentiation 223 or CD223) antagonists, cluster of differentiation 276 (CD276 or B7-H3) antigen modulators, cluster of differentiation 47 (CD47) antagonists, cluster of differentiation 30 (CD30) modulators, cluster of differentiation 73 (CD73) modulators, cluster of differentiation 66 (CD66) modulators, cluster of differentiation w137 (CDw137) agonists, cluster of differentiation 158 (CD158) modulators, cluster of differentiation 27 (CD27) modulators, cluster of differentiation 58 (CD58) modulators, cluster of differentiation 80 (CD80) modulators, cluster of differentiation 33 (CD33) modulators, cluster of differentiation 159 (CD159 or NKG2) modulators, glucocorticoid-induced TNFR-related (GITR) protein modulators, Killer Ig-like receptor (KIR) modulators, growth arrest-specific protein 6 (GAS6)/AXL pathway modulators, A proliferation-inducing ligand (APRIL) receptor modulators, human leukocyte antigen (HLA) modulators, epidermal growth factor receptor (EGFR) modulators, B-lymphocyte cell adhesion molecule modulators, cluster of differentiation w123 (CDw123) modulators, Erbb2 tyrosine kinase receptor modulators, endoglin modulators, mucin modulators, mesothelin modulators, hepatitis A virus cellular receptor 2 (HAVCR2) antagonists, cancer-testis antigen (CTA) modulators, tumor necrosis factor receptor superfamily, member 4 (TNFRSF4 or OX40) modulators, adenosine receptor modulators, inducible T cell co-stimulator (ICOS) modulators, cluster of differentiation 40 (CD40) modulators, tumor-infiltrating lymphocytes (TIL) therapies, or T-cell receptor (TCR) therapies.

Exemplary drugs that could be included in the wearable medicament delivery device package assemblies described herein include, but are not limited to: etanercept, abatacept, adalimumab, evolocumab, exenatide, secukinumab, erenumab, galcanezumab, fremanezumab-vfrm, alirocumab, methotrexate (amethopterin), tocilizumab, interferon beta-1a, interferon beta-1b, peginterferon beta-1a, sumatriptan, darbepoetin alfa, belimumab, sarilumab, semaglutide, dupilumab, reslizumab, omalizumab, glucagon, epinephrine, naloxone, insulin, amylin, vedolizumab, eculizumab, ravulizumab, crizanlizumab-tmca, certolizumab pegol, satralizumab, denosumab, romosozumab, benralizumab, emicizumab, tildrakizumab, ocrelizumab, ofatumumab, natalizumab, mepolizumab, risankizumab-rzaa, ixekizumab, and immune globulins.

Exemplary drugs that could be included in the wearable medicament delivery device package assemblies described herein may also include, but are not limited to, oncology treatments such as ipilimumab, nivolumab, pembrolizumab, atezolizumab, durvalumab, avelumab, cemiplimab, rituximab, trastuzumab, ado-trastuzumab emtansine, fam-trastuzumab deruxtecan-nxki, pertuzumab, transtuzumab-pertuzumab, alemtuzumab, belantamab mafodotin-blmf, bevacizumab, blinatumomab, brentuximab vedotin, cetuximab, daratumumab, elotuzumab, gemtuzumab ozogamicin, 90-Yttrium-ibritumomab tiuxetan, isatuximab, mogamulizumab, moxetumomab pasudotox, obinutuzumab, ofatumumab, olaratumab, panitumumab, polatuzumab vedotin, ramucirumab, sacituzumab govitecan, tafasitamab, or margetuximab.

Exemplary drugs that could be included in the wearable medicament delivery device package assemblies described herein include "generic" or biosimilar equivalents of any of the foregoing, and the foregoing molecular names should not be construed as limiting to the "innovator" or "branded" version of each, as in the non-limiting example of innovator medicament adalimumab and biosimilars such as adalimumab-afzb, adalimumab-atto, adalimumab-adbm, and adalimumab-adaz.

Exemplary drugs that could be included in the wearable medicament delivery device package assemblies described herein also include, but are not limited to, those used for adjuvant or neoadjuvant chemotherapy, such as an alkylating agent, plant alkaloid, antitumor antibiotic, antimetabolite, or topoisomerase inhibitor, enzyme, retinoid, or corticosteroid. Exemplary chemotherapy drugs include, by way of example but not limitation, 5-fluorouracil, cisplatin, carboplatin, oxaliplatin, doxorubicin, daunorubicin, idarubicin, epirubicin, paclitaxel, docetaxel, cyclophosphamide, ifosfamide, azacitidine, decitabine, bendamustine, bleomycin, bortezomib, busulfan, cabazitaxel, carmustine, cladribine, cytarabine, dacarbazine, etoposide, fludarabine, gemcitabine, irinotecan, leucovorin, melphalan, methotrexate, pemetrexed, mitomycin, mitoxantrone, temsirolimus, topotecan, valrubicin, vincristine, vinblastine, or vinorelbine.

Exemplary drugs that could be included in the wearable medicament delivery device package assemblies described herein also include, but are not limited to, analgesics (e.g., acetaminophen), antipyretics, corticosteroids (e.g. hydrocortisone, dexamethasone, or methylprednisolone), antihistamines (e.g., diphenhydramine or famotidine), antiemetics (e.g., ondansetron), antibiotics, antiseptics, anticoagulants, fibrinolytics (e.g., recombinant tissue plasminogen activator [r-TPA]), antithrombolytics, or diluents such as sterile water for injection (SWFI), 0.9% Normal Saline, 0.45% normal saline, 5% dextrose in water, 5% dextrose in 0.45% normal saline, Lactated Ringer's solution, Heparin Lock Flush solution, 100 U/mL Heparin Lock Flush Solution, or 5000 U/mL Heparin Lock Flush Solution.

Pharmaceutical formulations including, but not limited to, any drug described herein are also contemplated for use in the wearable medicament delivery device package assemblies described herein, for example pharmaceutical formulations comprising a drug as listed herein (or a pharmaceutically acceptable salt of the drug) and a pharmaceutically acceptable carrier. Such formulations may include one or more other active ingredients (e.g., as a combination of one or more active drugs), or may be the only active ingredient present, and may also include separately administered or co-formulated dispersion enhancers (e.g. an animal-derived, human-derived, or recombinant hyaluronidase enzyme), concentration modifiers or enhancers, stabilizers, buffers, or other excipients.

Exemplary drugs that could be included in the wearable medicament delivery device package assemblies described herein include, but are not limited to, a multi-medication treatment regimen such as AC, Dose-Dense AC, TCH, GT, EC, TAC, TC, TCHP, CMF, FOLFOX, mFOLFOX6, mFOLFOX7, FOLFCIS, CapeOx, FLOT, DCF, FOLFIRI, FOLFIRINOX, FOLFOXIRI, IROX, CHOP, R-CHOP, RCHOP-21, Mini-CHOP, Maxi-CHOP, VR-CAP, Dose-Dense CHOP, EPOCH, Dose-Adjusted EPOCH, R-EPOCH, CODOX-M, IVAC, HyperCVAD, R-HyperCVAD, SC-EPOCH-RR, DHAP, ESHAP, GDP, ICE, MINE, CEPP, CDOP, GemOx, CEOP, CEPP, CHOEP, CHP, GCVP, DHAX, CALGB 8811, HIDAC, MOpAD, 7 + 3, 5 +2, 7 + 4, MEC, CVP, RBAC500, DHA-Cis, DHA-Ca, DHA-Ox, RCVP, RCEPP, RCEOP, CMV, DDMVAC, GemFLP, ITP, VIDE, VDC, VAI, VDC-IE, MAP, PCV, FCR, FR, PCR, HDMP, OFAR, EMA/CO, EMA/EP, EP/EMA, TP/TE, BEP, TIP, VIP, TPEx, ABVD, BEACOPP, AVD, Mini-BEAM, IGEV, C-MOPP, GCD, GEMOX, CAV, DT-PACE, VTD-PACE, DCEP, ATG, VAC, VeIP, OFF, GTX, CAV, AD, MAID, AIM, VAC-IE, ADOC, or PE.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A wearable medicament delivery device package assembly (1, 101, 201) comprising:
a package body (10, 110, 210) defining an interior reservoir (12, 112, 212) for accommodating at least one medicament container (20),
a pressure generating module (30) configured to expel medicament from the medicament container (20),
a display (40, 140, 240) configured to present a message (50) with symbols (51) including letters (51a) and/or numbers (51b), the display (40) being configured to present the symbols (51) with a default predetermined symbol representation (53) and a control unit (60);
**characterised in that**
the control unit (60) is configured to receive a mirror indicating signal (62),
and in response of receiving the mirror indicating signal (62), instruct the display (40) to present the symbols (51) of the message (50) mirrored (55) relative to the default predetermined symbol representation (53).

2. The wearable medicament delivery device package assembly (1, 101, 201) according to claim 1, wherein the package body (10, 110, 210) comprises an outer surface (14, 114, 214), and wherein the display (40, 140, 240) is comprised in, or attached to, the outer surface (14, 114, 214).

3. The wearable medicament delivery device package assembly (1, 101, 201) according to any one of claims 1-2, wherein the display (40, 140, 240) is arranged to face away from the package body (10, 110, 210).

4. The wearable medicament delivery device package assembly (1, 101) according to any one of the preceding claims, further comprising a transceiver (70, 170) configured to detect an external mirror (1000) of the wearable medicament delivery device package assembly (1, 101), and in response of detecting the external mirror (1000), transmit the mirror indicating signal (62) to the control unit (60).

5. The wearable medicament delivery device package assembly (1, 101) according to claim 4, wherein the transceiver (70, 170) is configured to determine the degree of reflection of an external surface (1001), and wherein the external surface (1001) is detected as an external mirror (1000) in response to that the degree of reflection is above a predetermined threshold value.

6. The wearable medicament delivery device package assembly (1, 101) according to any one of claims 4-5, wherein the transceiver (70) is configured to continuously or periodically scan the surroundings for external mirrors (1000), or wherein the transceiver (170) is configured to scan the surroundings for external mirrors (1000) in response to receiving an external scanning signal, e.g. provided by a user of the wearable medicament delivery device package assembly (101).

7. The wearable medicament delivery device package assembly (201) according to any one of the preceding claims, further comprising a flip cover (216) having an internal mirror (218, 218'), the flip cover (216) being arrangeable in a folded position (P1) in which the flip cover (216) is arranged to cover the display (240), and being arrangeable in an unfolded position (P2) in which the flip cover (216) is arranged to at least partly uncover the display (240) such that at least a part of the internal mirror (218, 218') is distant from, and facing, the display (240), wherein the control unit (60) is configured to identify the unfolded position (P2) of the flip cover (216) as a mirror indicating signal (62).

8. The wearable medicament delivery device package assembly (201) according to claim 7, wherein the flip cover (216), in the unfolded position (P2), is arranged relative the display with an angle (a) of between 10° and 170°.

9. The wearable medicament delivery device package assembly (201) according to any one of claims 7-8, wherein the flip cover (216) extends from first end (216a) defined by a first end axis (A1), to a second end (216b) defined by a second end axis (A2), and wherein the flip cover (216) is attached to the package body (210) at the first end (216a) such that the flip cover (216) is foldable relative to the package body (210) by rotating about the first end axis (A1).

10. The wearable medicament delivery device package assembly (201) according to claim 9, wherein the flip cover (216) comprises a planar base portion (219) extending from the first end (216a) to the second end (216b), the planar base portion (219) having a first planar surface (219a) and a second planar surface (219b) arranged on the opposite side of planar base portion (219) as compared to the first planar surface (219a), wherein the internal mirror (219) is comprised in, or attached to, the first planar surface (219a).

11. The wearable medicament delivery device package assembly (201) according to claim 10, wherein the internal mirror (218) is provided as a mirrored coating on the first planar surface (219a).

12. The wearable medicament delivery device package assembly (201) according to any one of claims 10-11, wherein the internal mirror (218) is a one-way mirror and the planar base portion (219) is at least partly transparent.

13. The wearable medicament delivery device package assembly (201) according to any one of claims 7-12, wherein the internal mirror (218') is a concave mirror.

14. The wearable medicament delivery device package assembly (1, 101, 201) according to any one of the preceding claims, further comprising a wearable strap (90) attached to the package body (10, 110, 210), wherein the wearable strap (90) is configured to be worn around the user such that the package body (10, 110, 210) is arranged at a fixed position relative the user.

15. The wearable medicament delivery device package assembly (1, 101, 201) according to claim 14, wherein the display (40, 140, 240) is directly, or indirectly, attached to the wearable strap (90) such that the display (40, 140, 240) is arranged to face away from the user when the wearable medicament delivery device package assembly (1, 101, 201) is worn by the user.

## Patentansprüche

1. Verpackungsanordnung (1, 101, 201) für eine tragbare Arzneimittelabgabevorrichtung, umfassend:
einen Verpackungskörper (10, 110, 210), der ein inneres Reservoir (12, 112, 212) zum Aufnehmen von mindestens einem Arzneimittelbehälter (20) definiert,
ein Druckerzeugungsmodul (30), das konfiguriert ist, um ein Arzneimittel aus dem Arzneimittelbehälter (20) auszustoßen,
eine Anzeige (40, 140, 240), die konfiguriert ist, um eine Nachricht (50) mit Symbolen (51) einschließlich Buchstaben (51a) und/oder Zahlen (51b) darzustellen, wobei die Anzeige (40) konfiguriert ist, um die Symbole (51) mit einer standardmäßigen vorbestimmten Symboldarstellung (53) darzustellen, und
eine Steuereinheit (60); **dadurch gekennzeichnet, dass** die Steuereinheit (60) konfiguriert ist, um ein Spiegelangabesignal (62) zu empfangen,
und als Reaktion auf das Empfangen des Spiegelangabesignals (62) die Anzeige (40) anzuweisen, die Symbole (51) der Nachricht (50) relativ zu der standardmäßigen vorbestimmten Symboldarstellung (53) gespiegelt (55) darzustellen.

2. Verpackungsanordnung (1, 101, 201) für die tragbare Arzneimittelabgabevorrichtung nach Anspruch 1, wobei der Verpackungskörper (10, 110, 210) eine Außenoberfläche (14, 114, 214) umfasst und wobei die Anzeige (40, 140, 240) in der Außenoberfläche (14, 114, 214) umfasst oder an dieser angebracht ist.

3. Verpackungsanordnung (1, 101, 201) für die tragbare Arzneimittelabgabevorrichtung nach einem der Ansprüche 1 bis 2, wobei die Anzeige (40, 140, 240) angeordnet ist, um von dem Verpackungskörper (10, 110, 210) abgewandt zu sein.

4. Verpackungsanordnung (1, 101) für die tragbare Arzneimittelabgabevorrichtung nach einem der vorstehenden Ansprüche, ferner umfassend einen Sendeempfänger (70, 170), der konfiguriert ist, um einen externen Spiegel (1000) der Verpackungsanordnung für die tragbare Arzneimittelabgabevorrichtung (1, 101) zu erfassen und als Reaktion auf das Erfassen des externen Spiegels (1000) das Spiegelangabesignal (62) an die Steuereinheit (60) zu übertragen.

5. Verpackungsanordnung (1, 101) für die tragbare Arzneimittelabgabevorrichtung nach Anspruch 4, wobei der Sendeempfänger (70, 170) konfiguriert ist, um den Reflexionsgrad einer externen Oberfläche (1001) zu bestimmen, und wobei die externe Oberfläche (1001) als Reaktion darauf, dass der Reflexionsgrad über einem vorbestimmten Grenzwert liegt, als ein externer Spiegel (1000) erfasst wird.

6. Verpackungsanordnung (1, 101) für die tragbare Arzneimittelabgabevorrichtung nach einem der Ansprüche 4 bis 5, wobei der Sendeempfänger (70) konfiguriert ist, um die Umgebung kontinuierlich oder periodisch nach externen Spiegeln (1000) abzutasten, oder wobei der Sendeempfänger (170) konfiguriert ist, um die Umgebung als Reaktion auf das Empfangen eines externen Abtastsignals, z. B. durch einen Benutzer der Verpackungsanordnung (101) für die tragbare Arzneimittelabgabevorrichtung bereitgestellt, nach externen Spiegeln (1000) abzutasten.

7. Verpackungsanordnung (201) für die tragbare Arzneimittelabgabevorrichtung nach einem der vorstehenden Ansprüche, ferner umfassend eine Klappabdeckung (216), die einen Innenspiegel (218, 218') aufweist, wobei die Klappabdeckung (216) in einer gefalteten Position (P1) angeordnet werden kann, in der die Klappabdeckung (216) angeordnet ist, um die Anzeige (240) abzudecken, und in einer entfalteten Position (P2) angeordnet werden kann, in der die Klappabdeckung (216) angeordnet ist, um die Anzeige (240) mindestens teilweise aufzudecken, derart dass mindestens ein Teil des Innenspiegels (218, 218') von der Anzeige (240) beabstandet und dieser zugewandt ist, wobei die Steuereinheit (60) konfiguriert ist, um die entfaltete Position (P2) der Klappabdeckung (216) als ein Spiegelangabesignal (62) zu identifizieren.

8. Verpackungsanordnung (201) für die tragbare Arzneimittelabgabevorrichtung nach Anspruch 7, wobei die Klappabdeckung (216) in der entfalteten Position (P2) relativ zu der Anzeige mit einem Winkel (a) zwischen 10° und 170° angeordnet ist.

9. Verpackungsanordnung (201) für die tragbare Arzneimittelabgabevorrichtung nach einem der Ansprüche 7 bis 8, wobei sich die Klappabdeckung (216) von einem ersten Ende (216a), das durch eine erste Endachse (A1) definiert ist, zu einem zweiten Ende (216b), das durch eine zweite Endachse (A2) definiert ist, erstreckt und wobei die Klappabdeckung (216) derart an dem Verpackungskörper (210) an dem ersten Ende (216a) angebracht ist, dass die Klappabdeckung (216) relativ zu dem Verpackungskörper (210) durch ein Drehen um die erste Endachse (A1) herum faltbar ist.

10. Verpackungsanordnung (201) für die tragbare Arzneimittelabgabevorrichtung nach Anspruch 9, wobei die Klappabdeckung (216) einen planaren Basisabschnitt (219) umfasst, der sich von dem ersten Ende (216a) zu dem zweiten Ende (216b) erstreckt, wobei der planare Basisabschnitt (219) eine erste planare Oberfläche (219a) und eine zweite planare Oberfläche (219b) aufweist, die auf der gegenüberliegenden Seite des planaren Basisabschnitts (219) im Vergleich zu der ersten planaren Oberfläche (219a) angeordnet ist, wobei der Innenspiegel (219) in der ersten planaren Oberfläche (219a) umfasst oder an dieser angebracht ist.

11. Verpackungsanordnung (201) für die tragbare Arzneimittelabgabevorrichtung nach Anspruch 10, wobei der Innenspiegel (218) als eine gespiegelte Beschichtung auf der ersten planaren Oberfläche (219a) bereitgestellt ist.

12. Verpackungsanordnung (201) für die tragbare Arzneimittelabgabevorrichtung nach einem der Ansprüche 10 bis 11, wobei der Innenspiegel (218) ein halbdurchlässiger Spiegel ist und der planare Basisabschnitt (219) mindestens teilweise transparent ist.

13. Verpackungsanordnung (201) für die tragbare Arzneimittelabgabevorrichtung nach einem der Ansprüche 7 bis 12, wobei der Innenspiegel (218') ein konkaver Spiegel ist.

14. Verpackungsanordnung (1, 101, 201) für die tragbare Arzneimittelabgabevorrichtung nach einem der vorstehenden Ansprüche, ferner umfassend einen tragbaren Gurt (90), der an dem Verpackungskörper (10, 110, 210) angebracht ist, wobei der tragbare Gurt (90) konfiguriert ist, um derart um den Benutzer herum getragen zu werden, dass der Verpackungskörper (10, 110, 210) an einer festen Position relativ zu dem Benutzer angeordnet ist.

15. Verpackungsanordnung (1, 101, 201) für die tragbare Arzneimittelabgabevorrichtung nach Anspruch 14, wobei die Anzeige (40, 140, 240) derart direkt oder indirekt an dem tragbaren Gurt (90) angebracht ist, dass die Anzeige (40, 140, 240) angeordnet ist, um von dem Benutzer abgewandt zu sein, wenn Verpackungsanordnung (1, 101, 201) für die tragbare Arzneimittelabgabevorrichtung durch den Benutzer getragen wird.

## Revendications

1. Ensemble boîtier de dispositif de délivrance de médicament portable (1, 101, 201) comprenant :
un corps de boîtier (10, 110, 210) définissant un réservoir intérieur (12, 112, 212) permettant d'accueillir au moins un récipient de médicament (20),
un module de génération de pression (30) conçu pour expulser un médicament du récipient de médicament (20),
un affichage (40, 140, 240) configuré pour présenter un message (50) avec des symboles (51) comportant des lettres (51a) et/ou des chiffres (51b), l'affichage (40) étant configuré pour présenter les symboles (51) avec une représentation symbolique prédéterminée par défaut (53) et
une unité de commande (60) ; **caractérisé en ce que** l'unité de commande (60) est configurée pour recevoir un signal d'indication de miroir (62),
et en réponse à la réception du signal d'indication de miroir (62), donner pour instruction à l'affichage (40) de présenter les symboles (51) du message (50) en miroir (55) par rapport à la représentation symbolique prédéterminée par défaut (53).

2. Ensemble boîtier de dispositif de délivrance de médicament portable (1, 101, 201) selon la revendication 1, dans lequel le corps de boîtier (10, 110, 210) comprend une surface externe (14, 114, 214), et dans lequel l'affichage (40, 140, 240) est compris dans, ou fixé à, la surface externe (14, 114, 214).

3. Ensemble boîtier de dispositif de délivrance de médicament portable (1, 101, 201) selon l'une quelconque des revendications 1 à 2, dans lequel l'affichage (40, 140, 240) est agencé pour faire face à l'écart du corps de boîtier (10, 110, 210).

4. Ensemble boîtier de dispositif de délivrance de médicament portable (1, 101) selon l'une quelconque des revendications précédentes, comprenant en outre un transcepteur (70, 170) configuré pour détecter un miroir externe (1000) de l'ensemble boîtier de dispositif de délivrance de médicament portable (1, 101), et en réponse à la détection du miroir externe (1000), transmettre le signal d'indication de miroir (62) à l'unité de commande (60).

5. Ensemble boîtier de dispositif de délivrance de médicament portable (1, 101) selon la revendication 4, dans lequel le transcepteur (70, 170) est configuré pour déterminer le degré de réflexion d'une surface externe (1001), et dans lequel la surface externe (1001) est détectée en tant que miroir externe (1000) en réponse au fait que le degré de réflexion est supérieur à une valeur seuil prédéterminée.

6. Ensemble boîtier de dispositif de délivrance de médicament portable (1, 101) selon l'une quelconque des revendications 4 à 5, dans lequel le transcepteur (70) est configuré pour balayer de façon continue ou périodique les alentours à la recherche de miroirs externes (1000), ou dans lequel le transcepteur (170) est configuré pour balayer les alentours à la recherche de miroirs externes (1000) en réponse à la réception d'un signal de balayage externe, par exemple fourni par un utilisateur de l'ensemble boîtier de dispositif de délivrance de médicament portable (101).

7. Ensemble boîtier de dispositif de délivrance de médicament portable (201) selon l'une quelconque des revendications précédentes, comprenant en outre un couvercle rabattable (216) ayant un miroir interne (218, 218'), le couvercle rabattable (216) pouvant être agencé dans une position pliée (P1) dans laquelle le couvercle rabattable (216) est agencé pour couvrir l'affichage (240), et pouvant être agencé dans une position dépliée (P2) dans laquelle le couvercle rabattable (216) est agencé pour découvrir au moins partiellement l'affichage (240) de telle sorte qu'au moins une partie du miroir interne (218, 218') est distante de, et fait face vers, l'affichage (240), dans lequel l'unité de commande (60) est configurée pour identifier la position dépliée (P2) du couvercle rabattable (216) en tant que signal d'indication de miroir (62).

8. Ensemble boîtier de dispositif de délivrance de médicament portable (201) selon la revendication 7, dans lequel le couvercle rabattable (216), dans la position dépliée (P2), est agencé par rapport à l'affichage avec un angle (a) compris entre 10° et 170°.

9. Ensemble boîtier de dispositif de délivrance de médicament portable (201) selon l'une quelconque des revendications 7 à 8, dans lequel le couvercle rabattable (216) s'étend d'une première extrémité (216a) définie par un axe de première extrémité (A1), à une seconde extrémité (216b) définie par un axe de seconde extrémité (A2), et dans lequel le couvercle rabattable (216) est fixé au corps de boîtier (210) au niveau de la première extrémité (216a) de telle sorte que le couvercle rabattable (216) est pliable par rapport au corps de boîtier (210) par rotation autour de l'axe de première extrémité (A1).

10. Ensemble boîtier de dispositif de délivrance de médicament portable (201) selon la revendication 9, dans lequel le couvercle rabattable (216) comprend une partie de base plane (219) s'étendant de la première extrémité (216a) à la seconde extrémité (216b), la partie de base plane (219) ayant une première surface plane (219a) et une seconde surface plane (219b) agencée sur le côté opposé de la partie de base plane (219) par comparaison avec la première surface plane (219a), dans lequel le miroir interne (219) est compris dans, ou fixé à, la première surface plane (219a).

11. Ensemble boîtier de dispositif de délivrance de médicament portable (201) selon la revendication 10, dans lequel le miroir interne (218) est fourni en tant que revêtement miroité sur la première surface plane (219a).

12. Ensemble boîtier de dispositif de délivrance de médicament portable (201) selon l'une quelconque des revendications 10 à 11, dans lequel le miroir interne (218) est un miroir unidirectionnel et la partie de base plane (219) est au moins partiellement transparente.

13. Ensemble boîtier de dispositif de délivrance de médicament portable (201) selon l'une quelconque des revendications 7 à 12, dans lequel le miroir interne (218') est un miroir concave.

14. Ensemble boîtier de dispositif de délivrance de médicament portable (1, 101, 201) selon l'une quelconque des revendications précédentes, comprenant en outre une sangle portable (90) fixée au corps de boîtier (10, 110, 210), dans lequel la sangle portable (90) est conçue pour être portée autour de l'utilisateur de telle sorte que le corps de boîtier (10, 110, 210) est agencé à une position fixe par rapport à l'utilisateur.

15. Ensemble boîtier de dispositif de délivrance de médicament portable (1, 101,201) selon la revendication 14, dans lequel l'affichage (40, 140,240) est fixé directement, ou indirectement, à la sangle portable (90) de telle sorte que l'affichage (40, 140, 240) est agencé pour faire face à l'écart de l'utilisateur lorsque l'ensemble boîtier de dispositif de délivrance de médicament portable (1, 101, 201) est porté par l'utilisateur.
